(19) Europäisches Patentamt / European Patent Office / Office européen des brevets

(11) **EP 2 281 504 A1**

(12) **EUROPEAN PATENT APPLICATION**

(43) Date of publication:
**09.02.2011 Bulletin 2011/06**

(51) Int Cl.:
*A61B 5/00* (2006.01)    *A61B 5/021* (2006.01)
*A61B 5/024* (2006.01)    *A61B 5/029* (2006.01)

(21) Application number: **09167137.0**

(22) Date of filing: **04.08.2009**

(84) Designated Contracting States:
**AT BE BG CH CY CZ DE DK EE ES FI FR GB GR HR HU IE IS IT LI LT LU LV MC MK MT NL NO PL PT RO SE SI SK SM TR**
Designated Extension States:
**AL BA RS**

(71) Applicant: **Pulsion Medical Systems AG**
**81829 München (DE)**

(72) Inventor: **Joeken, Stefan, Dr.**
**79839, Lörrach (DE)**

(74) Representative: **Ettmayr, Andreas et al**
**Kehl & Ettmayr**
**Patentanwälte**
**Friedrich-Herschel-Straße 9**
**81679 München (DE)**

(54) **Apparatus and method for determining a physiological parameter**

(57)    An apparatus for determining a physiological parameter of a patient, such as cardiac output, comprises a sensor device providing readings of a blood variable, memory means storing said readings as a curve over time, evaluation means determining a mean value from the curve and determining the physiological parameter using the mean value is provided. The sensor device includes a plethysmography and/or pulse-oximetry sensor setup. The evaluation means determine a spectral density of said curve and/or a variance. The evaluation means also determine at least one model parameter representing an effective value of a heart beat using the mean value and said spectral density and/or variance. The effective value is selected from an effective amplitude of the heart beat, an effective duration of the heart beat and an effective area under the heart beat. The physiological parameter is determined using at least one of the model parameters.

Figure 1

## Description

The present invention relates to an apparatus for determining at least one physiological parameter of a patient. In particular, the invention relates to an apparatus for determining at least one physiological parameter of a patient, which comprises at least one sensor device adapted to provide readings of a blood variable of the patient, storage means for storing said readings as at least one curve representing said blood variable over time and evaluation means adapted to determine a mean value from said curve and to determine said at least one physiological parameter using said mean value.

In addition, the invention relates to a method for determining at least one physiological parameter of a patient by providing readings of a blood variable of the patient, storing said readings as at least one curve representing said blood variable over time determining a mean value from said pressure curve and determining said at least one physiological parameter using said mean value.

Furthermore, the invention relates to a physical storage medium having stored thereon a computer program which, when executed on a computer system, causes the computer system to perform a method for determining at least one physiological parameter of a patient.

Devices and methods for determining cardiac function parameters such as cardiac output, ejection fractions or stroke volume are well known in the state of the art. Specifically, cardiac output is obtainable from continuous blood pressure waveforms, which are derived by conventional means via an arterial line measuring the arterial blood pressure. Generally, the cardiac output is equal to the stroke volume multiplied by the heart rate.

For example, EP-A-0 920 278 describes a method for the measurement of cardiac output in a patient, which involves the continuous measurement of the arterial blood pressure waveform, then running an autocorrelation of the waveform data and finally establishing the cardiac output by multiplying the transformed autocorrelation data with a calibration factor using a known accurate calibration method for that patient.

EP-A-0 947 941 describes in vivo determination of the compliance function and the cardiac output of a patient using pulse contour analysis.

However, the current reliable methods for continuously determining cardiac output and/or stroke volume require calibration to a reference value, which usually is determined by an additional discontinuous measurement. In addition, pulse contour analyses require the reliable detection of the dicrotic notch to separate the systolic from the diastolic pulse pressure components. Furthermore, in some circumstances, such as during major surgery, the waveform may not display a dicrotic notch at all. In addition, the above approaches are compromised by disturbances in the pressure measurement due to arrhythmia or reflections from the arterial branches.

Further, there is a general desire to reduce the degree of invasiveness in hemodynamic measurement procedures.

Therefore, it is an object of the present invention to provide an apparatus or method, respectively, of the type described above for measuring a physiological parameter such as cardiac output, ejection fractions or stroke volume, which negotiates the necessity for the calibration of the measurement data with a reference value obtained by an additional discontinuous measurement for the patient.

A further object of the present invention is to provide an apparatus or method, respectively, of the type described above, which does away with the requirement to detect the dicrotic notch.

Additionally, it is a particular object of the current invention to minimize the said measurement disturbances in an apparatus or method as described above due to arrhythmia or reflections from the arterial branches.

It is further an object to decrease the degree of invasiveness involved in determining the physiological parameter (s).

According to one aspect of the present invention, these objects are achieved by an apparatus of the type initially mentioned,

wherein the sensor device includes a plethysmography and/or pulse-oximetry sensor setup and
wherein the evaluation means of the apparatus are further adapted

- to determine at least one of a spectral density $S(\omega)$ of the curve and a variance of the arterial blood variable,
- to determine at least one model parameter representing an effective value of a heart beat using the determined mean value and at least one of said spectral density $S(\omega)$ and said variance, said effective value being selected from an effective amplitude $A_{eff}$ of said heart beat, an effective duration $d_{eff}$ of said heart beat and an effective area $F_{eff}$ under said heart beat, and
- to determine the at least one physiological parameter using at least one said model parameter.

The present invention is thus carried out using an (opto-)plethysmography and/or pulse-oximetry sensor setup and basing the evaluation techniques on the plethysmography and/or pulse-oximetry readings, which usually include a plurality of intensity signals.

It is to be understood that the curve may directly be derived from raw data such as an electrical signal depending on a local light transmission and/or reflection detected by an optical sensor arrangement.

As (opto-)plethysmography and/or pulse-oximetry sensor setups can easily be implemented using non-invasive sensors (e.g. finger clip sensors, ear clip sensors or sensors affixed to the forehead), the present invention thus allows to greatly decrease or eliminate the invasiveness of physiological, in particular hemodynamic, parameter determination.

(Opto-)plethysmography and pulse-oximetry sensors per se are widely known from the prior art. Plethysmography is a technique for measuring blood volume variations correlating with a subject's pulse. This technique allows implementation of continuous non-invasive pulse frequency measurements, as well as measurements of local blood volume/perfusion, which are also indicative for blood pressure conditions. Pulse-oximetry measurements allow determining oxygen saturation of the blood. In optoplethysmography there are mainly two alternative measurement principles: transmission measurements and reflexion measurements. Transmission measurements are based on measurement of intensity according to Lambert-Beer Law. In optoplethysmography usually two or more wavelengths are used within the range of approximately 600 nm through approximately 1000 nm, usually about 660 nm and 940 nm. Suitable measurement sites are, e.g., fingertips, ear lobes, forehead (for reflexion measurements), nose and toes. Technical sensor implementations have been widely published both in patent and non-patent literature, such as Vincent Chan, Steve Underwood: "A Single-Chip Pulsoximeter Design Using the MSP430" SLAA274 - November 2005 (http://focus.ti.com/lit/an/slaa274/slaa274.pdf) and Texas Instruments Medical Applications Guide 1Q2009, p. 37-44 (http://focus.ti.com/lit/an/slyb108d/slyb108d.pdf).

Preferably, said at least one physiological parameter includes at least one of stroke volume SV, cardiac output CO and ejection fraction EF.

It is further preferred that said evaluation means are adapted to determine said stroke volume SV as a product of a first model parameter representing said effective amplitude $A_{eff}$ and a second model parameter representing said effective duration $d_{eff}$.

In addition, it is preferred that said evaluation means are adapted to determine said ejection fraction EF as a product of a model parameter representing said effective duration $d_{eff}$ and a heart rate HR of said patient.

In particular preferred is that said evaluation means are adapted to determine said cardiac output CO as a product of a first model parameter representing said effective amplitude $A_{eff}$, a second model parameter representing said effective duration $d_{eff}$ and an approximation of a heart rate of said patient.

Preferably, said approximation of said heart rate is selected from an actual measured heart rate HR and an approximate function $\lambda_{eff}$, said approximate function $\lambda_{eff}$ including a quotient with a dividend comprising the square of the mean value and a divisor comprising the spectral density $S(\omega)$ at $\omega=0$.

Further preferably, said evaluation means are adapted to use a correction parameter $\alpha$ in determining said model parameter, said correction parameter $\alpha$ assuming values greater than or equal to 1, said values being the higher, the less the patient's heart frequency deviates from a rhythmic condition.

In addition, it is preferred that said evaluation means are adapted to use a monotonous correction function $\sigma$ depending on said correction parameter $\alpha$ and assuming values from 0 to 1, wherein said correction function $\sigma$ assumes the value of 0 if said correction parameter $\alpha$ equals 1 and said correction function $\sigma$ assumes the value of 1 for said correction parameter $\alpha$ tending to infinity.

Preferably, said effective amplitude $A_{eff}$ is provided as a quotient with a dividend comprising the sum of said variance and the product of said correction function $\sigma$ and the square of the mean value and a divisor comprising the mean value.

Furthermore preferably, said effective amplitude $d_{eff}$ is provided as a quotient with a dividend comprising the spectral density $S(\omega)$ at $\omega=0$ and a divisor comprising the sum of said variance and the product of said correction function $\sigma$ and the square of the mean value.

Also preferably, said apparatus provides Fourier Transformation means for determining said spectral density $S(\omega)$ as the Fourier Transformation of the autocorrelation of said curve.

Preferably, the spectral density $S(\omega)$ at $\omega=0$ is provided as said variance multiplied by a constant factor.

In an additional preferable embodiment said apparatus further comprises

- means for administering a travelling deviation of an intrinsic physical quantity (such as temperature, an optical/spectral property or an indicator concentration) to the blood stream of said patient at a first location of the blood circulation of said patient, and
- sensor means for measuring said physical quantity at a second location of the blood circulation of said patient over the course of time,

wherein, said memory means are adapted to record said physical quantity measured over the course of time at said second location as a dilution curve, and said evaluation unit is adapted to determine a comparative value of at least one of said physiological parameters from said dilution curve using dilution algorithms.

Preferably, said evaluation unit is adapted to use said comparative value for calibration.

Preferably, said evaluation unit is further adapted to determine a blood oxygen saturation value from said readings and to output said blood oxygen saturation value.

Preferably, said calibration includes determining, using said comparative value, a correction parameter $\alpha$ used in determining said model parameter, said correction parameter $\alpha$ assuming values greater than or equal to 1, said values being the higher the less the patient's heart frequency deviates from a rhythmic condition.

In a preferred embodiment, said evaluation unit is adapted to reject and re-calculate said physiological parameter, if the difference between the determined physiological parameter and the respective comparative value exceeds a threshold value.

Further to these embodiments, the above objects are achieved under an additional aspect of the invention by a method for determining at least one physiological parameter of a patient, said method comprising the steps of:

- importing readings of a blood variable of said patient,
- storing said readings as a curve representing said blood variable over time t,
- determining a mean value from said curve and determining at least one physiological parameter using said mean value

wherein said imported readings are readings obtained through plethysmographic and/or pulsoximetric measurement techniques and

wherein the method further includes

- determining at least one of a spectral density $S(\omega)$ of said curve and a variance of said blood variable,
- determining at least one model parameter representing an effective value of a heart beat using said mean value and at least one of said spectral density $S(\omega)$ and said variance, said effective value being selected from an effective amplitude $A_{eff}$ of said heart beat, an effective duration $d_{eff}$ of said heart beat and an effective area $F_{eff}$ under said heart beat, and
- determining said at least one physiological parameter using at least one said model parameter.

Preferably, in above method said at least one physiological parameter includes at least one of stroke volume SV, cardiac output CO and ejection fraction EF.

Furthermore preferably in above method, said stroke volume SV is determined as a product of a first model parameter representing said effective amplitude $A_{eff}$ and a second model parameter representing said effective duration $d_{eff}$.

It is further preferred that in above method said ejection fraction EF is determined as a product of a model parameter representing said effective duration $d_{eff}$ and a heart rate HR of said patient.

Further preferably, in above method said cardiac output CO is determined as a product of a first model parameter representing said effective amplitude $A_{eff}$, a second model parameter representing said effective duration $d_{eff}$ and an approximation of a heart rate of said patient.

Also preferably in above method, said approximation of said heart rate is selected from an actual measured heart rate HR and an approximate function $\lambda_{eff}$, said approximate function $\lambda_{eff}$ including a quotient with a dividend comprising the square of the mean value and a divisor comprising the spectral density $S(\omega)$ at $\omega = 0$.

In addition, in above method, it is preferred that a correction parameter $\alpha$ is used in determining said model parameter, said correction parameter $\alpha$ assuming values greater than or equal to 1, said values being the higher the less the patient's heart frequency deviates from a rhythmic condition.

Preferably, in above method, a monotonous correction function $\sigma$ is used depending on said correction parameter $\alpha$ and assuming values from 0 to 1, wherein said correction function $\sigma$ assumes the value of 0 if said correction parameter $\alpha$ equals 1 and said correction function $\sigma$ assumes the value of 1 for said correction parameter $\alpha$ tending to infinity.

In a preferred embodiment of above method, said effective amplitude $A_{eff}$ is provided as a quotient, the dividend of said quotient comprising the sum of said variance and the product of said correction function $\sigma$ and the square of the mean value and the divisor of said quotient comprising the mean value.

Preferably, in above method, said effective amplitude $d_{eff}$ is provided as a quotient with a dividend comprising the spectral density $S(\omega)$ at $\omega = 0$ and a divisor comprising the sum of said variance and the product of said correction function $\sigma$ and the square of the mean value.

It is further preferred that in above method, said spectral density $S(\omega)$ is determined as the Fourier Transformation of the autocorrelation of said curve.

Preferably, in above method, the spectral density $S(\omega)$ at $\omega = 0$ is provided as said variance multiplied by a constant factor.

In a preferred embodiment, above method further comprises

- inputting information about a travelling deviation of an intrinsic physical quantity (such as temperature, an optical/ spectral property or an indicator concentration) administered to the blood stream of said patient at a first location of the blood circulation of said patient, and
- reading in measurement readings of said physical quantity at a second location of the blood circulation of said patient over the course of time,

wherein, said measurement readings of said physical quantity at said second location of the blood circulation of said patient are recorded as a dilution curve, and said comparative value of at least one of said physiological parameters is determined from said dilution curve using dilution algorithms.

Preferably, in above method, said comparative value is used for calibration.

Preferably, in above method said evaluation unit is used for determining a blood oxygen saturation value from said readings and for outputting said blood oxygen saturation value.

In addition in above method, it is preferred that said calibration includes determining, using said comparative value, a correction parameter $\alpha$ used in determining said model parameter, said correction parameter $\alpha$ assuming values greater than or equal to 1, said values being the higher the less the patient's heart frequency deviates from a rhythmic condition.

Preferably, in above method, said physiological parameter is rejected and recalculated, if the difference between the determined physiological parameter and the respective comparative value exceeds a threshold value.

An additional embodiment of the present invention comprises a physical storage medium having stored thereon a computer program which, when executed on a computer system, causes the computer system to perform a method as described above.

Generally, any of the embodiments described or options mentioned herein may be particularly advantageous depending on the actual conditions of application. Further, features of one embodiment may be combined with features of another embodiment as well as features known per se from the prior art as far as technically possible and unless indicated otherwise.

In the following, the invention is described in more detail. The accompanying drawings, which are schematic flow diagrams and graphs, serve for a better understanding of the features of the current invention.

Therein:

Fig. 1    is a flow diagram illustrating a preferred embodiment of the inventive method for determining the cardiac output CO. Optional features are presented in dashed lines. The quantity $\alpha$ may depend on the CVP (central venous pressure) and/or the heart rate variability,

Fig. 2    is an exemplary plot of the functional relationship $\sigma(\alpha)$, i.e. the behaviour of $\sigma$ for typical values of $\alpha$, according to one embodiment of the present invention

Fig. 3    shows a correlation plot between the CO values determined according to an embodiment of the present invention by eq. (24) and the reference values $CO_{ref}$ obtained by the transpulmonary cardiac output measurements.

Fig. 4    illustrates the general setup of an apparatus according to an embodiment of the present invention making use of an optoplethysmography sensor.

Fig. 5    shows a modified setup similar to fig. 4.

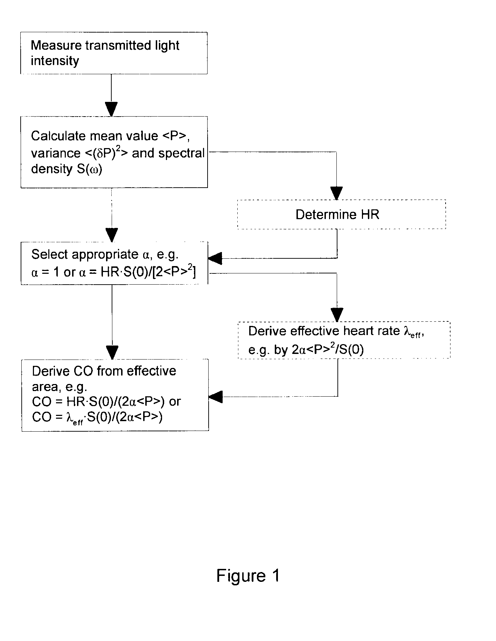

Figure 1 illustrates (indicated by regular lines) the most important steps of one preferred embodiment of the present invention. Dashed lines indicate optional steps for modifying the embodiment to yield another preferred embodiment. In order to improve the illustration of the preferred embodiments and to ease the understanding thereof, the following description also attempts to describe in more detail the underlying calculatory principles.

While the main focus of the described embodiments is on cardiac output determination, other physiological parameters may also be established with the apparatus and methods according to the invention, in particular, such parameters characterising cardiac function, such as ejection fractions or stroke volume.

As is shown in figure 1, the current invention makes use of measurement readings representing the *transmitted light intensity, the readings being provided either as raw data (such* as *voltage or electric current) from a suitable sensor or already in a pre-processed state.*

As already mentioned above, plethysmography is a technique for measuring blood volume variations correlating with a subject's pulse and thus arterial blood pressure. This technique also allows implementation of continuous pulse frequency measurements. Further, pulse-oximetry measurements allow determining oxygen saturation of the blood.

Arteries of the vascular system are flexible and therefore expand depending on the blood pressure during the systole. Consequently, since the plethysmography or pulse-oximetry readings are pressure-dependent, the readings relate to an arterial blood pressure and the curve representing said variable over time corresponds, to a certain degree, to a pressure curve over time t. Likewise, the mean arterial blood pressure is a corresponding variable to said mean value. Therefore, in the following, the blood variable is represented by the character P for being analogous to a measured pressure.

The blood variable P depends on time $t$ and results from the superposition of several heart beats $b_k(t - t_k)$, beginning at time $t_k$. Due to reflections in the arterial tree, several heart beats may contribute to the resulting blood variable, which can be described by the following function:

$$P(t) = \sum_k b_k(t - t_k) \qquad (1)$$

In general, all beats may differ from each other, however, for a limited period of time, an average beat $b(t)$ exists so that the function of equation (1) can be described adequately by

$$P(t) = \sum_k b_k(t - t_k) \qquad (2)$$

$$\approx \sum_k b(t - t_k) \qquad (3)$$

As in healthy living beings the heart rate HR usually does not vary strongly within a limited time frame (e.g. 10 s to 200 s), successive beats are almost equidistant and the occurrence of beats is determined by $t_k = k/HR$.

During strong arrhythmia, however, heart beats occur very irregularly. The worst case scenario is the random occurrence of independent beats, which can be described by a Poisson-Process, i.e. the intervals between successive heart beats are distributed at random according to an exponential-distribution $q$ such that:

$$q(t_k - t_{k-1}) = HR * exp(-HR * (t_k - t_{k-1})).$$

In this case, the superposition of equation (3) is a Campbell-Process, as is described in: [N. R. Campbell, "The study of discontinuous phenomena." Proc. Camp. Philos. Soc. Math. Phys. Sci. 15:117-136, 1909.]

According to the present embodiment, the cardiac function can be assessed in both conditions, i.e. for regular and irregular heart activity, by means of the mean, variance and spectral density of the curve representing the blood variable over time $P(t)$,

The mean value $<P>$ which essentially corresponds to a mean assessed pressure is derived from the integral of the curve representing the blood variable over time $P(t)$ over an appropriate time span $T$, i.e.

$$<P> = \frac{1}{T} \int_0^T P(t)dt \qquad (4)$$

The Variance of the blood variable is calculated from $\delta P(t) = P(t) - <P>$ so that:

$$< (\delta P(t))^2 > \;\; = \;\; \frac{1}{T} \int_0^T (P(t) - <P>)^2 dt \tag{5}$$

The Spectral density is the Fourier Transformation of the autocorrelation of *P(t)* and hence is determined by:

$$S(\omega) \;\; = \;\; 4 \int_0^\infty \cos(\omega\tau) < \delta P(t) * \delta P(t - \tau) > d\tau \tag{6}$$

In the case of arrhythmia, the arrhythmic conditions can be described by a Campbell-Process application of equations (4) to (6), which results in the following functions:

$$<P> \;\; = \;\; HR * \int_0^\infty b(t)dt \tag{7}$$

$$< (\delta P)^2 > \;\; = \;\; HR * \int_0^\infty b^2(t)dt \tag{8}$$

$$S(\omega) \;\; = \;\; 2 * HR * \left| \tilde{b}(\omega) \right|^2 \tag{9}$$

with the Fourier Transformation $\tilde{b}(\omega)$ of the average beat *b(t):*

$$\tilde{b}(\omega) \;\; = \;\; \int_0^\infty b(t) * e^{-i\omega t} dt \tag{10}$$

For rhythmic conditions with periodic heart beats, the application of equations (4) to (6) results to:

$$<P> \;\; = \;\; HR * \int_0^\infty b(t)dt \tag{11}$$

$$< (\delta P)^2 > \;\; = \;\; HR * \int_0^\infty b^2(t)dt \; - \; <P>^2 \tag{12}$$

$$S(\omega) = 2\alpha * HR * \left|\tilde{b}(\omega)\right|^2, \quad \text{with} \quad \alpha > 1 \qquad (13)$$

The parameter $\alpha$ depends on the heart rate variability on a time scale much larger than T. For practical implementations, $\alpha$ can be determined through emperical studies or simply estimated taking into account the principles described herein.

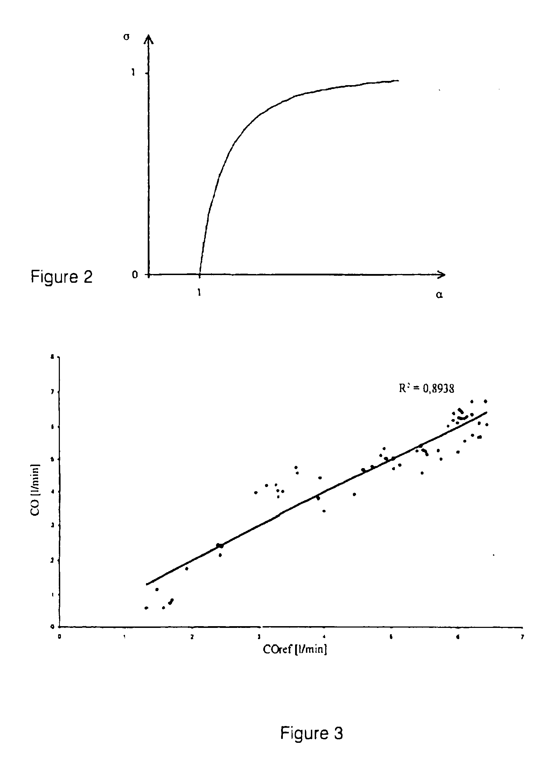

Merging the different conditions can be based on the following considerations. Equations (7) to (9) and (11) to (13) have the same structure. In particular, $\alpha$ in eq. (13) decreases with a decreasing accuracy in the periodicity of the beats *b(t)*. Finally, for randomly occurring beats, $\alpha$ tends to the lower limit $\alpha = 1$ and the equations (13) and (11) become identical. Equations (12) and (8) can also be merged. Therefore, a function $\sigma(\alpha)$ is introduced, which tends to 0 as $\alpha$ tends to 1, and $\sigma(\alpha)$ tends to 1 for $\alpha \gg 1$. This relationship is described in an exemplary manner in figure 2.

In consequence, the rhythmic, arrhythmic and intermediate activity of the heart is described by:

$$<P> = HR * \int_0^\infty b(t)dt \qquad (14)$$

$$<(\delta P)^2> = HR * \int_0^\infty b^2(t)dt - \sigma(\alpha)<P>^2 \qquad (15)$$

$$S(\omega) = 2\alpha * HR * \left|\tilde{b}(\omega)\right|^2 \qquad (16)$$

wherein $\alpha$ and $\sigma$ are limited as follows:

|  | $\alpha$ | $\sigma$ (alpha) |
|---|---|---|
| arrhythmic | 1 | 0 |
| intermediate | $\geq 1$ | > 0 and < 1 |
| rhythmic | >1 | 1 |

Furthermore, the acquired data may be interpreted so as to afford a characterisation of the average beat in terms of its amplitude *A* and duration *d*.

$$A := \frac{\int b^2(t)dt}{\int b(t)dt} \qquad (17)$$

$$d := \frac{\left(\int b(t)dt\right)^2}{\int b^2(t)dt} \qquad (18)$$

Thus, if a square wave is assumed,

$$b(t) = A, \qquad for\ 0 \le t \le d$$
$$b(t) = 0, \qquad otherwise \qquad (19)$$

Consequently, introducing the equations (14) to (16) yields:

$$A_{eff} = \frac{<(\delta P)^2> + \sigma <P>^2}{<P>} \qquad (20)$$

$$d_{eff} = \frac{S(0)}{2\alpha*(<(\delta P)^2> + \sigma <P>^2)} \qquad (21)$$

Therein, the index "eff" was introduced to point out that $A_{eff}$ and $d_{eff}$ are effective values reconstructed from the curve representing the blood variable over time $P(t)$. It is emphasized that this reconstruction is possible in all conditions, even if no heart beats are detectable in the curve $P(t)$. Moreover, the product of the area $A_{eff}$ and duration $d_{eff}$ results in the area under the effective beat $F_{eff}$, which is

$$F_{eff} = A_{eff} * d_{eff}$$
$$= \frac{S(0)}{2\alpha <P>} \qquad (22)$$

even if the beats do not represent a square wave.
In addition, the ratio

$$\lambda_{eff} = \frac{2\alpha <P>^2}{S(0)} \qquad (23)$$

gives an approximation of the heart rate in all circumstances.
According to the present embodiments, the obtained parameters are used to characterize a patient's cardiac function. In particular, the quantities $A_{eff}$, $d_{eff}$, $\lambda_{eff}$ and combinations thereof are useful to characterize stroke volume SV, cardiac output CO, ejection fraction EF and others.
Specifically, the Cardiac output CO may be derived according to

$$CO = HR * A_{eff} * d_{eff}$$

$$= HR * \frac{S(0)}{2\alpha <P>} \qquad (24)$$

In order to test the values obtained with the inventive method, a comparison with a known standard reference method was undertaken. For this purpose the cardiac output was measured according to the present invention and also with the established thermodilution technique according to EP-A-0 637 932.

The results of this comparison are displayed in figure 3. Therein, the abscissa represents cardiac output in liters per minute as determined by the reference method and the ordinate represents output in liters per minute as determined by the inventive method. Accordingly, the results achieved for the cardiac output in both measurements are in good agreement.

Furthermore, the other cardiac functions stroke volume and ejection fraction are also readily available from the parameters determined according to the inventive method.

The stroke volume SV is afforded by dividing the cardiac output by the heart rate HR; thus: $SV = A_{eff} * d_{eff}$.

Further, the quantity $d_{eff} * HR$ corresponds to the (left ventricular) ejection fraction EF.

Further embodiments of the invention will be described below.

An optional simplification to the inventive method may be made, if the spectral density is required for frequencies $f$ close to or at $\omega = 2\pi f = 0$. Then according to eq. (6), the variance of the blood variable $P$ can be used to approximate

$$S(0) \approx 2 * (<P^2> - <P>^2) \qquad (25)$$

$$= 2 * <(\delta P)^2> \qquad (26)$$

This simplification can also be used in the other relations, particularly, in eq. (24).

In addition, an arrhythmia adjustment may be made since the coefficient $1/\alpha$ may depend on the heart rate variability.

Furthermore, the central venous pressure CVP may influence the coefficient $1/\alpha$. Therefore, in order to establish $\alpha$, CVP can be taken into account either by reading in measurement data directly as provided by a CVP measurement source or by user input. Generally, measuring CVP per se is well known from the prior art and not part of the inventive method.

The effect of a varying heart rate on the current invention can be detected by determining the number of heart beats within a pressure sample and the effective heart rate $\lambda_{eff}$ determined by equation (23). Particularly, the ratio of HR and $\lambda_{eff}$ leads to

$$\alpha = \frac{HR * S(0)}{2<P>^2} \qquad (27)$$

Therefore, an adjustment of a patient monitoring system implementing the present invention to the current heart rate variability is possible.

The Spectral density S(0) for $\omega = 2\pi f\ 0$ can also be determined differently, e.g.

a) by means of the spectral density for frequencies tending to zero, i.e. $S(0) = \lim_{\omega \to 0} S(\omega) = \lim_{f \to 0} S(2\pi f)$, or

b) by fitting an appropriate function (e.g. $S(\omega) = S(0) / [1 + (\omega\tau)^2]^{n+1}$) to the measured spectrum. Therein, local maxima corresponding to the heart rate should preferably be neglected.

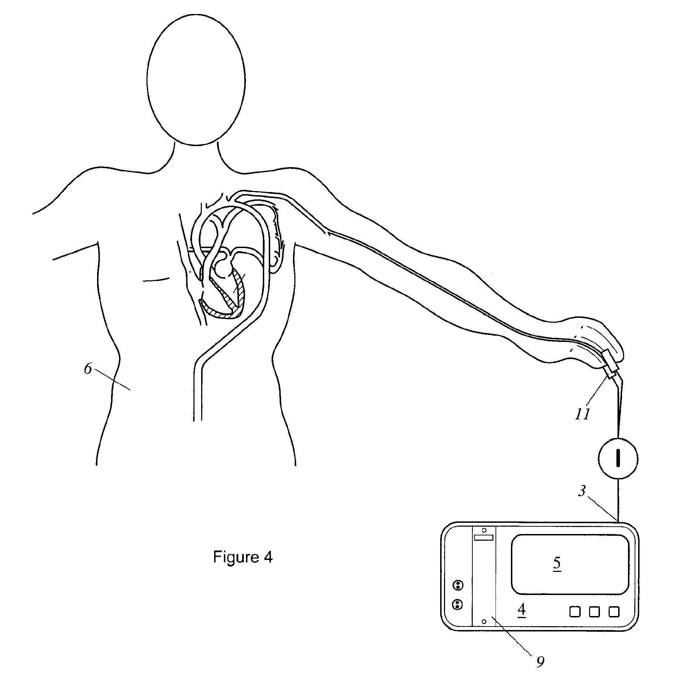

The embodiments described hereinabove in detail are based on plethysmographic measurements and/or on pulse-oximetry measurements as mentioned in the introductory part of this description. Fig. 4 shows a setup with an optoplethysmographic finger-clip sensor 11 employing a photometric means for measuring transmitted light intensity and transmitting a respective signal to the input channel 3 of the patient monitoring apparatus 4. Instead of a finger-clip sensor 11 other suitable sensor arrangements such as a an ear-lobe clip, toe-clip or strap-on sensor unit may also be used. The patient monitoring apparatus 4 contains appropriate storage means for storing the photometer readings over time and serving as a processing storage.

The patient monitoring also comprises a computing facility 9, which may include a digital signal processing instance 9, which is programmed to perform calculations in accordance with equations as described above, wherein, however, the curve and terms derived therefrom are substituted by respective terms based on the plethysmography measurement.

With reference to the procedure described in figure 1, a first blood variable P is determineded by said apparatus depicted in figure 4 by plethysmographic and/or pulsoximetric measurement techniques.

By means of equation (6), the computing facility 9 calculates the mean value, the variance and the spectral density for the mean value determined within an appropriate time frame (e.g. 10 s to 200 s). For calculating the spectral density, designated fast fourier transformation means may be employed.

Furthermore, for $\omega = 0$ and $\alpha = 1$ the computing facility 9 employs equation (22) to calculate the effective area $F_{eff}$. The relationship between the effective area $F_{eff}$ and the stroke volume SV is given by $1/\alpha$, wherein the latter can be determined empirically or alternatively, with respect to reference SV measurements (e.g. transpulmonary thermodilution) from the treated patient. In order to perform such reference SV measurements, the patient monitoring apparatus 4 may be equipped accordingly, e.g. by providing additional input channels for thermodilution measurement readings and by implementing suitable thermodilution algorithms in the computing facility 9, as known per se from the prior art.

The knowledge of SV permits the determination of CO according to *CO = HR * SV,* wherein the heart rate HR can also, optionally, be determined from the pressure and the spectral density or by other methods.

A display 5 to visualize the determined parameters (as numeric values, graphically or both) and control elements 10 to operate said apparatus are also provided. In addition, the determined parameters may be stored at a recording medium and/or printed. For this purpose, the patient monitoring apparatus 4 may comprise various interface ports for connecting peripheral equipment.

The embodiment of figure 4 is configured such that the actual sensor device is essentially integrated in the finger clip sensor 11, whereas the evaluation means are integrated in the monitoring apparatus 4. However, it is also possible to advantageously implement the invention in a manner wherein the evaluation means are essentially integrated in a finger clip unit, ear-lobe clip unit, toe clip unit, strap-on unit or the like that houses the sensor. In such a compact arrangement, the determined parameter(s) may be indicated on a small display on the sensor unit and/or be transmitted wireless or by cable to a patient monitoring apparatus.

Further, it is possible to implement the evaluation means partially in a clip or strap-on sensor unit and partially in a patient monitoring apparatus, wherein intermediate evaluation data is transmitted from the clip or strap-on sensor unit to the patient monitor. Such an arrangement will require that monitoring apparatus and sensor unit are particularly adapted to each other and will thus assure that in practice only fully compatible components are used. A potential risk of combining a non-validated sensor with the patient monitoring apparatus can, depending on the transmission protocol, be easily reduced or fully avoided.

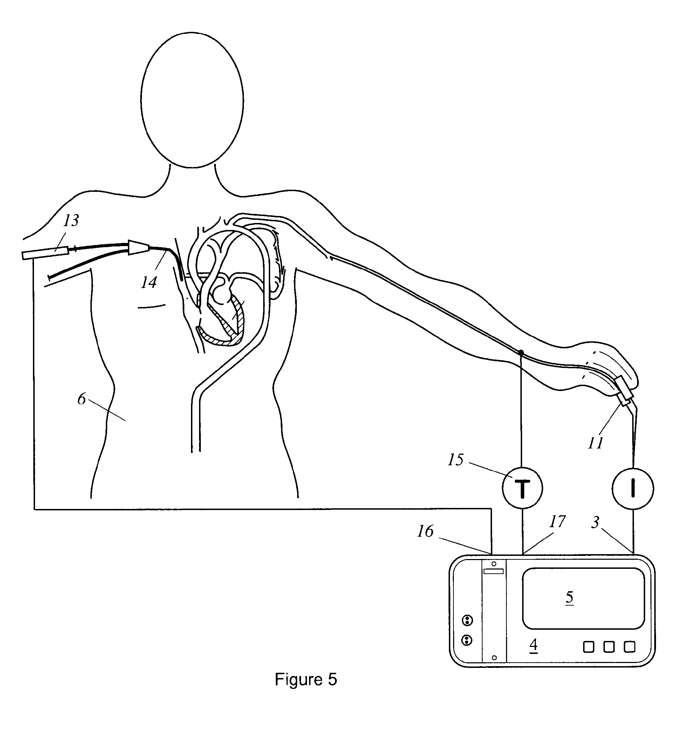

The embodiment depicted in figure 5 is generally set up as the embodiment of figure 4 but additionally comprises an injection channel 13 for administering a bolus of a temperature below blood temperature through a central venous catheter 14 to the blood stream in the vena cava sperior of the patient, and a temperature sensor 15 for measuring an arterial blood temperature over the course of time. Both the arterial blood temperature and a signal indicating temperature of the injected bolus and the time of injection are provided to the patient monitoring apparatus 4 via additional input channels 16 and 17, respectively. The memory means of the patient monitoring apparatus 4 record the arterial blood temperature measured over the course of time to determine a thermodilution curve therefrom. The patient monitoring apparatus 4 is adapted to determine, using thermodilution algorithms known per se, at least one comparative value of a physiological parameter from the dilution curve in order to allow calibration of the system.

**Claims**

1.  Apparatus for determining at least one physiological parameter of a patient, said apparatus comprising:

    - a sensor device adapted to provide readings of a blood variable of said patient,

- memory means for storing said readings as a curve representing said variable over time t,
- evaluation means adapted to determine a mean value from said curve and to determine said at least one physiological parameter using said mean value,

**characterized in that**
said sensor device includes a plethysmography and/or pulse-oximetry sensor setup and
said evaluation means are further adapted

- to determine at least one of a spectral density $S(\omega)$ of said curve and a variance of said blood variable,
- to determine at least one model parameter representing an effective value of a heart beat using said mean value and at least one of said spectral density $S(\omega)$ and said variance, said effective value being selected from an effective amplitude $A_{eff}$ of said heart beat, an effective duration $d_{eff}$ of said heart beat and an effective area $F_{eff}$ under said heart beat, and
- to determine said at least one physiological parameter using at least one said model parameter.

2. Apparatus according to claim 1, wherein said at least one physiological parameter includes at least one of stroke volume SV, cardiac output CO and ejection fraction EF.

3. Apparatus according to claim 2, wherein said evaluation means are adapted to determine said stroke volume SV as a product of a first model parameter representing said effective amplitude $A_{eff}$ and a second model parameter representing said effective duration $d_{eff}$.

4. Apparatus according to claim 2 or claim 3, wherein said evaluation means are adapted to determine said ejection fraction EF as a product of a model parameter representing said effective duration $d_{eff}$ and a heart rate HR of said patient.

5. Apparatus according to one of claims 2 to 4, wherein said evaluation means are adapted to determine said cardiac output CO as a product of a first model parameter representing said effective amplitude $A_{eff}$, a second model parameter representing said effective duration $d_{eff}$ and an approximation of a heart rate of said patient.

6. Apparatus according to claim 5, wherein said approximation of said heart rate is selected from an actual measured heart rate HR and an approximate function $\lambda_{eff}$, said approximate function $\lambda_{eff}$ including a quotient with a dividend comprising the square of the mean value and a divisor comprising the spectral density $S(\omega)$ at $\omega=0$.

7. Apparatus according to any of the preceding claims, wherein said evaluation means are adapted to use a correction parameter $\alpha$ in determining said model parameter, said correction parameter $\alpha$ assuming values greater than or equal to 1, said values being the higher, the less the patient's heart frequency deviates from a rhythmic condition.

8. Apparatus according to claim 7, wherein said evaluation means are adapted to use a monotonous correction function $\sigma$ depending on said correction parameter $\alpha$ and assuming values from 0 to 1, wherein said correction function $\sigma$ assumes the value of 0 if said correction parameter $\alpha$ equals 1 and said correction function $\sigma$ assumes the value of 1 for said correction parameter $\alpha$ tending to infinity.

9. Apparatus according to claim 8, wherein said effective amplitude $A_{eff}$ is provided as a quotient with a dividend comprising the sum of said variance and the product of said correction function $\sigma$ and the square of the mean value and a divisor comprising the mean value.

10. Apparatus according to claim 8 or claim 9, wherein said effective amplitude $d_{eff}$ is provided as a quotient with a dividend comprising the spectral density $S(\omega)$ at $\omega=0$ and a divisor comprising the sum of said variance and the product of said correction function $\sigma$ and the square of the mean value.

11. Apparatus according to any of the preceding claims comprising Fourier Transformation means for determining said spectral density $S(\omega)$ as the Fourier Transformation of the autocorrelation of said curve.

12. Apparatus according to any of the preceding claims, wherein the spectral density $S(\omega)$ at $\omega=0$ is provided as said variance multiplied by a constant factor.

13. Apparatus according to any of the preceding claims, further comprising

- means for administering a travelling deviation of an intrinsic physical quantity to the blood stream of said patient at a first location of the blood circulation of said patient, and
- sensor means for measuring said physical quantity at a second location of the blood circulation of said patient over the course of time,

wherein said memory means are adapted to record said physical quantity measured over the course of time at said second location as a dilution curve, and said evaluation unit is adapted to determine a comparative value of at least one of said physiological parameters from said dilution curve using dilution algorithms.

14. Apparatus according to any of the preceding claims, wherein said evaluation unit is further adapted to determine a blood oxygen saturation value from said readings and to output said blood oxygen saturation value.

15. Method for determining at least one physiological parameter of a patient, said method comprising the steps of:

- importing readings of a blood variable of said patient,
- storing said readings as a curve representing said blood variable over time t,
- determining a mean value from said curve and determining at least one physiological parameter using said mean value

**characterized in that**
said imported readings are readings obtained through plethysmographic and/or pulsoximetric measurement techniques and
said method further includes

- determining at least one of a spectral density $S(\omega)$ of said curve and a variance of said blood variable,
- determining at least one model parameter representing an effective value of a heart beat using said mean value and at least one of said spectral density $S(\omega)$ and said variance, said effective value being selected from an effective amplitude $A_{eff}$ of said heart beat, an effective duration $d_{eff}$ of said heart beat and an effective area $F_{eff}$ under said heart beat, and
- determining said at least one physiological parameter using at least one said model parameter.

Figure 1

Figure 2

Figure 3

Figure 4

Figure 5

Europäisches
Patentamt

European
Patent Office

Office européen
des brevets

**EUROPEAN SEARCH REPORT**

Application Number

EP 09 16 7137

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A,E | EP 2 087 836 A (PULSION MEDICAL SYS AG [DE]) 12 August 2009 (2009-08-12) * the whole document * ----- | 1-15 | INV. A61B5/00 A61B5/021 A61B5/024 A61B5/029 |
| A | WO 2005/055825 A (EDWARDS LIFESCIENCES CORP [US]; ROTELIUK LUCHY [US]) 23 June 2005 (2005-06-23) * paragraph [0035] * * paragraph [0042] - paragraph [0044] * * paragraph [0072] - paragraph [0076] * * paragraph [0089] - paragraph [0090] * ----- | 1,15 | |
| A | WO 2008/019207 A (EDWARDS LIFESCIENCES CORP [US]; HATIB FERAS S [US]; MOONEY CHARLES R []) 14 February 2008 (2008-02-14) * paragraph [0021] * * paragraph [0069] - paragraph [0071] * * paragraph [0111] * * figure 11 * ----- | 1,15 | |
| A | EP 1 870 032 A (GEN ELECTRIC [US]) 26 December 2007 (2007-12-26) * paragraph [0024] * * paragraph [0030] * * paragraph [0032] * ----- | 1,15 | TECHNICAL FIELDS SEARCHED (IPC) A61B |

The present search report has been drawn up for all claims

| Place of search | Date of completion of the search | Examiner |
|---|---|---|
| Munich | 16 November 2009 | Gärtner, Andreas |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**ANNEX TO THE EUROPEAN SEARCH REPORT
ON EUROPEAN PATENT APPLICATION NO.**                      EP 09 16 7137

This annex lists the patent family members relating to the patent documents cited in the above-mentioned European search report.
The members are as contained in the European Patent Office EDP file on
The European Patent Office is in no way liable for these particulars which are merely given for the purpose of information.

16-11-2009

| Patent document cited in search report | | Publication date | Patent family member(s) | | Publication date |
|---|---|---|---|---|---|
| EP 2087836 | A | 12-08-2009 | BR | PI0805530 A2 | 06-10-2009 |
| | | | CN | 101502414 A | 12-08-2009 |
| | | | JP | 2009183715 A | 20-08-2009 |
| | | | US | 2009204012 A1 | 13-08-2009 |
| WO 2005055825 | A | 23-06-2005 | BR | PI0416542 A | 09-01-2007 |
| | | | CA | 2548592 A1 | 23-06-2005 |
| | | | EP | 1689294 A1 | 16-08-2006 |
| | | | JP | 2007512921 T | 24-05-2007 |
| | | | US | 2005187481 A1 | 25-08-2005 |
| WO 2008019207 | A | 14-02-2008 | NONE | | |
| EP 1870032 | A | 26-12-2007 | US | 2008009753 A1 | 10-01-2008 |

EPO FORM P0459

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 0920278 A
- EP 0947941 A
- EP 0637932 A

**Non-patent literature cited in the description**

- **Vincent Chan ; Steve Underwood.** A Single-Chip Pulsoximeter Design Using the MSP430. *SLAA274,* November 2005
- Texas Instruments Medical Applications Guide 1Q2009. 37-44
- **N. R. Campbell.** The study of discontinuous phenomena. *Proc. Camp. Philos. Soc. Math. Phys. Sci.,* 1909, vol. 15, 117-136